# EUROPEAN PATENT SPECIFICATION

(11) **EP 3 006 927 B1**
(45) Date of publication and mention of the grant of the patent: **02.08.2017**
(21) Application number: 15188421.0
(22) Date of filing: 05.10.2015
(51) Int. Cl.: G01N 27/16, G01N 33/00

(54) **SYSTEM WITH CATALYST BASED CO SENSOR AND METHOD FOR PROTECTING THE SENSOR AGAINST SULFUR POISON AND COKE DEPOSITION**
SYSTEM MIT KATALYSATORBASIERTEM CO-SENSOR UND VERFAHREN ZUM SCHUTZ DES SENSORS GEGEN SCHWEFELGIFT UND KOKSABLAGERUNG
SYSTÈME À CAPTEUR DE CO À BASE DE CATALYSEUR ET PROCÉDÉ POUR PROTÉGER LE CAPTEUR CONTRE L'EMPOISONNEMENT AU SOUFRE ET UN DÉPÔT DE COKE

(30) Priority: 09.10.2014 CN 201410527677
(43) Date of publication of application: 13.04.2016
(73) Proprietor: General Electric Company, Schenectady, NY 12345 (US)
(72) Inventor: ZHANG, Minglong, Shanghai 31 201203 (CN); MOYEDA, David K, Irvine, CA 92614 (US); ZHONG, Dalong, Shanghai 201203 (CN); NAREDDY, Santosh K., Irvine, CA 92614 (US); CHANG, Hai, Shanghai 31 201203 (CN); CHEN, Pei, Shanghai 31 201203 (CN)
(74) Representative: Foster, Christopher Michael

(56) References cited:
- EP-A1- 1 720 008
- JP-A- H08 226 909
- JP-A- 2001 004 587
- JP-A- 2008 014 662

## Description

### BACKGROUND

A fossil fuel station burns fossil fuels such as coal, natural gas or petroleum to produce electricity. The thermal energy of combustion is converted into mechanical energy, which then operates an electrical generator. The level of carbon monoxide equivalent (COe) in the flue gas from the combustion is a reliable, accurate and sensitive indication of unburned combustibles, wherein carbon monoxide equivalent refers to combustible gases including not only carbon monoxide but also other combustibles such as hydrogen, methane, ethane and other higher hydrocarbon components. Thus the COe level in the flue gas is monitored to control the air-fuel ratio in order to achieve optimal combustion efficiency. Based on different technologies and principles, several devices, such as solid state CO sensors, and optical detectors including infrared (IR), non-dispersed infrared (NDIR) and tunable diode laser spectrometers, can be used to measure the COe level in flue gas from a combustion boiler. COe trim control then assures minimal energy loss and maximum efficiency, independent of boiler load, fuel type, or fuel quantity.

One method is to use a catalyst based CO sensor to measure the COe level. The CO sensor uses an oxidation catalyst to oxidize COe into carbon dioxide (CO₂). During the catalytic oxidation reaction, reaction heat is generated to increase the temperature. The temperature increase is measured and used to estimate the COe level in the flue gas. However, the catalyst may be deteriorated quickly in the presence of some harmful materials. For example, the CO sensor may fail due to formation of sulfate solid products on the sensor surface in the presence of sulfur oxides existing in the flue gas. Coke deposition on the CO sensor surface is observed in some applications as well.

Thus, there is a need for a fuel combustion system in which a catalyst based CO sensor for detecting a COe level is protected from being quickly deteriorated by sulfur poison and coke deposition.

JP 2001 004857 A describes a potentiometric sensor for detecting carbon monoxide comprising platinum electrodes mounted on a solid electrolyte and covered with a gas selective permeation layer, which acts as a filter for absorbing sulfur dioxide.

JP H08 226909 A describes a catalytic combustion type CO sensor comprising a catalyst containing platinum coated on an alumina support formed round a platinum wire RTD.

### BRIEF DESCRIPTION

In one aspect, a carbon monoxide (CO) sensor device is provided according to claim 1. The sampling device is adapted for sampling flue gas and includes a sulfur oxide absorber for absorbing sulfur oxides in the sampled flue gas. The CO sensor is coupled to the sampling device for receiving the sampled flue gas after the sulfur oxides have been absorbed, and includes an active element including platinum.

In another aspect, a method for sampling flue gas from a fuel combustion boiler, absorbing sulfur oxides in the sampled flue gas, and then detecting a carbon monoxide equivalent level in the sampled flue gas by a CO sensor is recited in claim 10, whereby the CO sensor is operated at a temperature higher than both a sulfate decomposition temperature above which a sulfate decomposition rate is higher than a sulfate deposition rate and a carbon coke combustion temperature above which a carbon coke combustion rate is higher than a carbon coke deposition rate.

### BRIEF DESCRIPTION OF THE DRAWINGS

The above and other aspects, features, and advantages of the present disclosure will become more apparent in light of the subsequent detailed description when taken in conjunction with the accompanying drawing in which:
FIG. 1 is a schematic diagram illustrating an exemplary CO sensor device, according to one embodiment of the present disclosure.
FIG. 2 is a schematic diagram illustrating an exemplary CO sensor device, according to another embodiment of the present disclosure.

### DETAILED DESCRIPTION

One or more embodiments of the present disclosure will be described below. Unless defined otherwise, technical and scientific terms used herein have the same meaning as is commonly understood by one of ordinary skill in the art to which this invention belongs. The term "or" is meant to be inclusive and mean any, some, or all of the listed items. The use of "including," "comprising" or "having" and variations thereof herein are meant to encompass the items listed thereafter and equivalents thereof as well as additional items.

Approximating language, as used herein throughout the specification and claims, may be applied to modify any quantitative representation that could permissibly vary without resulting in a change in the basic function to which it is related. Accordingly, a value modified by a term or terms, such as "about" and "substantially", are not to be limited to the precise value specified. Additionally, when using an expression of "about a first value - a second value," the about is intended to modify both values. In at least some instances, the approximating language may correspond to the precision of an instrument for measuring the value. Here, and throughout the specification and claims, range limitations may be combined and/or interchanged. As for a fuel combustion boiler which burns a fuel/air mixture to produce electricity, it is desired to control the air-fuel ratio at an ideal value to achieve the highest combustion efficiency. The air-fuel ratio can be regulated by monitoring the net oxygen (O₂) level and the carbon monoxide equivalent (COe) level in the flue gas from the combustion boiler and adjusting combustions conditions to drive the ratio towards the ideal value. A CO sensor is arranged at a downstream side of the combustion boiler to detect the COe level in the flue gas from the combustion boiler.

The CO sensor includes a catalyst which can facilitate an exothermic reaction of COe and then cause a temperature change. In one example the catalyst comprises a platinum (Pt) containing catalyst. In some embodiments, a catalyst based CO sensor used for detecting the COe level includes an active element and a non-active reference, which are active and non-active for the oxidization to convert COe into carbon dioxide (CO₂), respectively. In one specific embodiment, the active element comprises a Pt/Al₂O₃ coated resistance temperature detector (RTD), and the non-active reference comprises an Al₂O₃ coated RTD. The Pt functions as an oxidation catalyst, and the Al₂O₃ is a very good heat conductor. The catalytic oxidation reaction generates heat on the active element, the temperature of which is measured against the non-active reference. The COe level in the flue gas can be estimated from a temperature difference between the active element and the non-active reference. Thus the Pt catalyst based sensor can in-situ detect the COe level in the flue gas quickly, which helps to tune the air-fuel ratio for achieving optimal combustion efficiency. The Pt/Al₂O₃ catalyst is particularly applicable for CO oxidation in high temperature and harsh environments. But the Pt/Al₂O₃ catalyst may deteriorate quickly in the presence of sulfur oxides in the flue gas from the combustion boiler. SO₂ may be oxidized to SO₃ by the Pt/Al₂O₃ catalyst and then SO₃ may react with Al₂O₃ to form Al₂(SO₄)₃ sulfate which is stable at temperatures below 500 degree Celsius. The formation of Al₂(SO₄)₃ sulfate on the sensor surface may degrade the efficiency of catalytic oxidation since it reduces the effective contact area of the Pt/Al₂O₃ catalyst with COe and O₂ gases. It is found when the SO₂ concentration is smaller than 400 ppm, the Pt/Al₂O₃ catalyst may lose about 10% efficiency and then become stable. Thus the catalyst is still effective on the catalytic oxidation of CO. However, when the SO₂ concentration exceeds about 400 ppm, especially when the SO₂ concentration exceeds about 500 ppm, the sulfate may be continuously formed on the catalyst surface, which may deteriorate the Pt/Al₂O₃ catalyst and degrade its catalytic capability, and finally may completely isolate the catalyst from gas and cause permanent failure of the CO sensor.

In order to improve the lifetime and reliability of a catalyst based CO sensor used in a fuel combustion system, two approaches may be utilized. One is to load a sulfur oxide absorber in the gas sampling device of the CO sensor to reduce sulfur oxides such as SO₂ and SO₃ in the flue gas to a safe level, for example 400 ppm, before the flue gas contacts the Pt/Al₂O₃ catalyst, to avoid the sulfur oxide poison effect on the catalyst. The sulfur oxide absorber may be selected from metal hydroxides, metal oxides, carbonates and combinations thereof. Some examples of the sulfur oxide absorber include CaCO₃, CaO, Ca(OH)₂, MgCO₃, MgO and Mg(OH)₂. Another approach is to operate the CO sensor at a higher temperature, for example 500 degree Celsius or more, to avoid carbon coke and sulfate deposition on a platinum containing surface of the CO sensor. In the invention, the two approaches are combined.

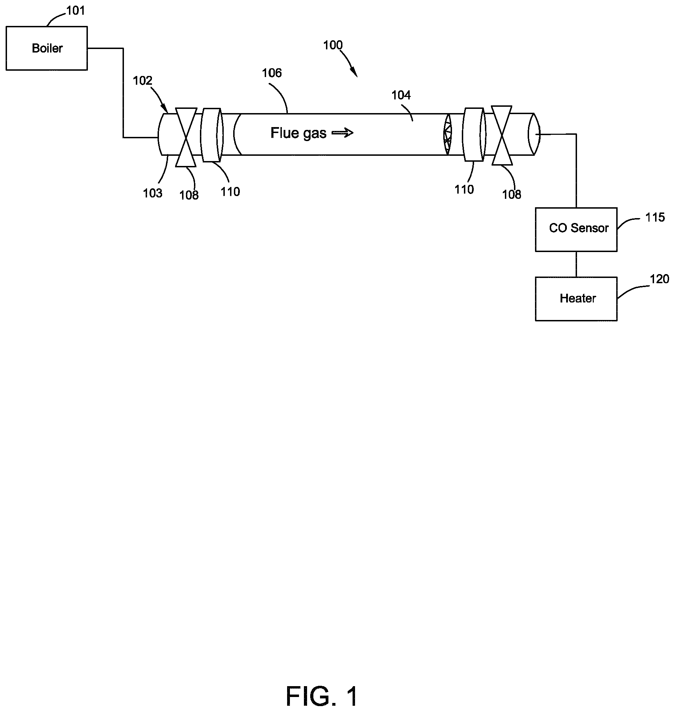

Referring to FIG. 1, a CO sensor device 100 of a fuel combustion system includes a sampling device 102 for sampling flue gas from a fossil fuel boiler 101. A sulfur oxide absorber 104 is arranged in a portion of the sampling device 102 for absorbing sulfur oxides, for example SO₂ and/or SO₃, in the sampled flue gas . A CO sensor 115 is coupled to the sampling device 102 for detecting the COe level in the sampled flue gas after the sulfur oxides have been absorbed. The CO sensor has an active element with a Pt containing coating, which may be deteriorated by sulfur oxides in the flue gas. There may be a heater 120 for controlling the operation temperature of the CO sensor 115 at an appropriate value.

The sampling device 102 may include a sampling conduit 103 for transferring a sampled flue gas to the CO sensor 115, and one or more sampling elements, for example, sample probes, pumps or valves, adapted for carrying out the sampling. The sulfur oxide absorber 104 may be arranged in the sampling conduit 103 directly, or in an additional container 106 connected to the sampling conduit 103, i.e., between two sections of the sampling conduit 103, by valves 108 and flanges 110. In the embodiment as illustrated in FIG. 1, the additional container 106 is a pipe with a radial size substantially identical with that of the sampling conduit 103. As the additional container 106 is detachably connected to the sampling conduit 103 by valves 108 and flanges 110, it can be installed easily and quickly, and also can be replaced without limitation from the hardware design. In other embodiments, the additional container 106 may be configured with other shapes and/or dimensions.

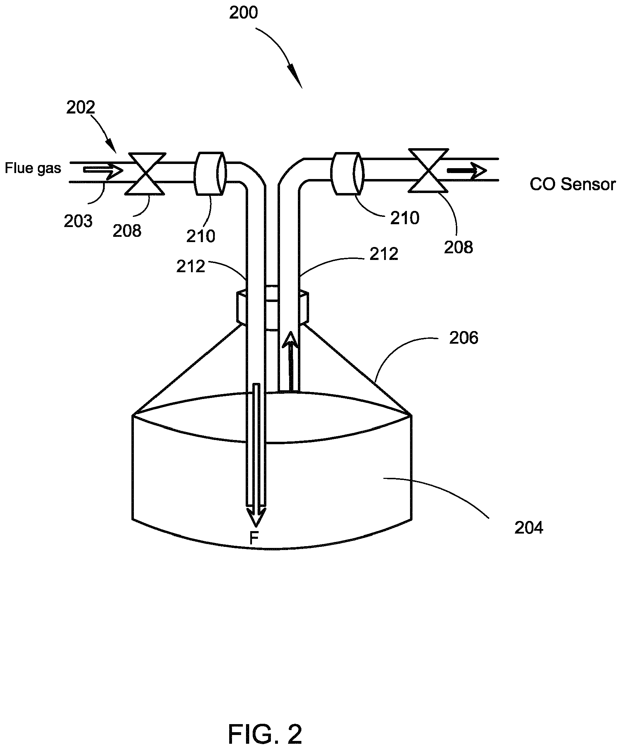

For example, a container 206 of another configuration is used in a CO sensor device 200 as illustrated in FIG. 2. Similar to the CO sensor device 100 of FIG. 1, in the CO sensor device 200, container 206 is connected to a sampling conduit 203 of a sampling device 202, by valves 208, flanges 210 and pipes 212. A sulfur oxide absorber 204 is loaded in the container 206, such that the sampled flue gas flows through the sulfur oxide absorber 204 to the CO sensor (not shown) on a downstream side of the container 206. Different from the container 106, the container 206 has a radial size larger than that of the sampling conduit 203. As used herein, the radial size refers to a size of the container in a direction substantially perpendicular to a flow direction F of the sampled flue gas passing through the container.

The sulfur oxide absorber 104 or 204 may be situated in a porous structure. Examples of porous structures include honeycomb, pipes and any others for high absorbing efficiency. In an exemplary embodiment, the sulfur oxide absorber is situated in a porous honeycomb structure.

The Pt catalyst on the CO sensor can completely oxidize from COe to CO₂ if there is enough oxygen adsorbed together with COe on the Pt/Al₂O₃ coating of the CO sensor. But in an abnormal environment, when the COe:O₂ ratio is too high and the adsorbed oxygen is not enough, and/or when the CO sensor is degraded, a disproportionate reaction of COe (e.g., 2CO=CO₂+C) may happen to cause coke formation. Moreover, hydrocarbon existing in the flue gas may also be incompletely oxidized to cause coke formation due to lack of enough oxygen. It is found when the COe concentration exceeds about 800 ppm, the adsorbed O₂ on the catalyst surface may become not enough, and the coke deposition may take a critical role on the CO sensor failure, especially when the catalyst is continuously exposed to flue gas of a high COe level.

Therefore, the sulfate deposition caused by the high SO₂ level and the carbon coke deposition caused by continuously exposing the CO sensor to a flue gas with a high COe level may be the root causes of a sensor failure. In some situations, levels of both SO₂ and COe are high and thus both sulfates, mainly Al₂(SO₄)₃ and coke deposition occur on the sensor surface. The sulfate and coke may accelerate the formation of each other. Finally, they may completely cover the catalyst surface and block the contact between catalyst and gases to make the CO sensor completely fail.

To avoid such a situation, the CO sensor is operated at a relatively higher temperature, at which the sulfates and carbon coke deposited on the sensor surface can be decomposed and combusted. Compared with conventional CO sensor devices in which the CO temperature is controlled at about 400 degree Celsius and the CO sensor temperature is operated at about 370 degree Celsius to keep the sampled gas above its dew point, the operation temperature of the CO sensor in the fuel combustion systems of the present disclosure is significantly increased. According to the invention, the CO sensor is operated at a temperature higher than both a sulfate decomposition temperature above which the sulfate decomposition rate is higher than the sulfate (e.g., Al₂(SO₄)₃) deposition rate and a carbon coke combustion temperature above which the carbon coke combustion rate is higher than the carbon coke deposition rate. In an exemplary embodiment, the CO sensor is operated at a temperature higher than about 500 degree Celsius, or preferably higher than about 550 degree Celsius. The operation temperature of the CO sensor is controlled at the appropriate value by a heater (like the heater 120 of FIG. 1).

By using a sulfur oxide absorber arranged in the gas sampling device of the CO sensor to reduce or remove sulfur compounds and/or operating the CO sensor at a relatively higher temperature at which the sulfates and carbon coke deposited on the sensor surface can be decomposed and combusted, the CO sensor is protected against sulfate poisoning and/or carbon coke deposition. Therefore the lifetime and reliability of the CO sensor is significantly improved. Moreover, both approaches can be implemented with minimal changes on the current hardware, software and operation.

The invention may be embodied in other specific forms. The foregoing embodiments are therefore to be considered in all respects as illustrative rather than limiting on the invention described herein. The scope of embodiments of the invention is thus indicated by the appended claims rather than by the foregoing description, and all changes that come within the meaning and range of equivalency of the claims are therefore intended to be embraced therein.

## Claims

1. A carbon monoxide (CO) sensor device (100), comprising:
a sampling device (102) for sampling flue gas, the sampling device (102) comprising a sulfur oxide absorber (104) for absorbing sulfur oxides in the sampled flue gas;
a CO sensor (115) coupled to the sampling device (102) for receiving the sampled flue gas after the sulfur oxides have been absorbed, the CO sensor (115) comprising an active element comprising platinum; and
a heater (120) adapted to control the CO sensor (115) at a temperature higher than both a sulfate decomposition temperature above which a sulfate decomposition rate is higher than a sulfate deposition rate and a carbon coke combustion temperature above which a carbon coke combustion rate is higher than a carbon coke deposition rate.

2. The CO sensor device according to claim 1, wherein the sulfur oxide absorber (104) is selected from metal hydroxides, metal oxides, carbonates and combinations thereof.

3. The CO sensor device according to claim 1 or 2, wherein the sampling device (102) comprises a conduit (103) for transferring the sampled flue gas to the CO sensor (115) and the sulfur oxide absorber (104) is arranged in the conduit (103).

4. The CO sensor device according to claim 1 or 2, wherein the sampling device (102) comprises a conduit (103) for transferring the sampled flue gas to the CO sensor (115) and the sulfur oxide absorber (104) is arranged in a container (106) connected between two sections of the conduit (103).

5. The CO sensor device according to claim 4, wherein the container (106) has a radial size substantially identical with that of the conduit (103) in a direction substantially perpendicular to a flow direction of the sampled flue gas.

6. The CO sensor device according to claim 4, wherein the container (206) has a radial size larger than that of the conduit (203) in a direction substantially perpendicular to a flow direction of the sampled flue gas.

7. The CO sensor device according to any preceding claim, wherein the sulfur oxide absorber (104) is situated in a porous structure.

8. The CO sensor device according to claim 7, wherein the sulfur oxide absorber (104) is situated in a honeycomb structure.

9. The CO sensor device according to any preceding claim, wherein the active element further comprises aluminium oxide.

10. A method, comprising:
sampling flue gas from a fuel combustion boiler (101);
absorbing sulfur oxides in the sampled flue gas; and then
detecting a carbon monoxide equivalent level in the sampled flue gas by a CO sensor (115) as described in claim 1, whereby the CO sensor (115) is operated at a temperature higher than both a sulfate decomposition temperature above which a sulfate decomposition rate is higher than a sulfate deposition rate and a carbon coke combustion temperature above which a carbon coke combustion rate is higher than a carbon coke deposition rate.

11. The method according to claim 10, wherein absorbing the sulfur oxide comprises using a sulfur oxide absorber (104) selected from metal hydroxides, metal oxides, carbonates and combinations thereof.

12. The method according to claim 11, wherein the sulfur oxide absorber (104) is situated in at least one of a porous structure or a honeycomb structure.

13. The method according to any of claims 10 to 12, wherein the active element further comprises aluminium oxide.

## Patentansprüche

1. Kohlenmonoxid- (CO)-Sensorvorrichtung (100), umfassend:
eine Beprobungsvorrichtung (102) zum Beproben von Abgas, wobei die Beprobungsvorrichtung (102) einen Schwefeloxidabsorber (104) zum Absorbieren von Schwefeloxiden in dem beprobten Abgas umfasst;
einen CO-Sensor (115), der an die Beprobungsvorrichtung (102) gekoppelt ist, um das beprobte Abgas aufzunehmen, nachdem die Schwefeloxide absorbiert wurden, wobei der CO-Sensor (115) ein aktives Element umfasst, welches Platin umfasst; und
einen Heizer (120), der adaptiert ist, um den CO-Sensor (115) auf eine Temperatur zu regeln, die höher als sowohl eine Sulfatzersetzungstemperatur als auch eine Kohlenstoffkoksverbrennungstemperatur ist, wobei oberhalb der Sulfatzersetzungstemperatur eine Sulfatzersetzungsrate höher als eine Sulfatabscheidungsrate ist und oberhalb einer Kohlenstoffkoksverbrennungstemperatur eine Kohlenstoffkoksverbrennungsrate höher als eine Kohlenstoffabscheidungsrate ist.

2. CO-Sensorvorrichtung nach Anspruch 1, wobei der Schwefeloxidabsorber (104) ausgewählt ist aus Metallhydroxiden, Metalloxiden, Carbonaten und Kombinationen davon.

3. CO-Sensorvorrichtung nach Anspruch 1 oder 2, wobei die Beprobungsvorrichtung (102) eine Rohrleitung (103) umfasst, um das beprobte Abgas zu dem CO-Sensor (115) zu transferieren, und wobei der Schwefeloxidabsorber (104) in der Rohrleitung (103) angeordnet ist.

4. CO-Sensorvorrichtung nach Anspruch 1 oder 2, wobei die Beprobungsvorrichtung (102) eine Rohrleitung (103) umfasst, um das beprobte Abgas zu dem CO-Sensor (115) zu transferieren, und wobei der Schwefeloxidabsorber (104) in einem Behälter (106) angeordnet ist, der zwischen zwei Abschnitten der Rohrleitung (103) angeschlossen ist.

5. CO-Sensorvorrichtung nach Anspruch 4, wobei der Behälter (106) eine radiale Größe aufweist, die in einer Richtung, die im Wesentlichen senkrecht zu einer Strömungsrichtung des beprobten Abgases ist, im Wesentlichen mit derjenigen der Rohrleitung (103) identisch ist.

6. CO-Sensorvorrichtung nach Anspruch 4, wobei der Behälter (206) eine radiale Größe aufweist, die in einer Richtung, die im Wesentlichen senkrecht zu einer Strömungsrichtung des beprobten Abgases ist, größer als diejenige der Rohrleitung (203) ist.

7. CO-Sensorvorrichtung nach einem der vorhergehenden Ansprüche, wobei der Schwefeloxidabsorber (104) sich in einer porösen Struktur befindet.

8. CO-Sensorvorrichtung nach Anspruch 7, wobei der Schwefeloxidabsorber (104) sich in einer Wabenstruktur befindet.

9. CO-Sensorvorrichtung nach einem der vorhergehenden Ansprüche, wobei das aktive Element ferner Aluminiumoxid umfasst.

10. Verfahren, umfassend:
Beproben von Abgas aus einem Brennstoffverbrennungskessel (101);
Absorbieren von Schwefeloxiden in dem beprobten Abgas; und danach
Detektieren eines Kohlenmonoxidäquivalentgehalts in dem beprobten Abgas durch einen CO-Sensor (115) wie in Anspruch 1 beschrieben, wobei der CO-Sensor (115) bei einer Temperatur betrieben wird, die höher als sowohl eine Sulfatzersetzungstemperatur als auch eine Kohlenstoffkoksverbrennungstemperatur ist, wobei oberhalb der Sulfatzersetzungstemperatur eine Sulfatzersetzungsrate höher als eine Sulfatabscheidungsrate ist und oberhalb einer Kohlenstoffkoksverbrennungstemperatur eine Kohlenstoffkoksverbrennungsrate höher als eine Kohlenstoffabscheidungsrate ist.

11. Verfahren nach Anspruch 10, wobei das Absorbieren des Schwefeloxids das Verwenden eines Schwefeloxidabsorbers (104) ausgewählt aus Metallhydroxiden, Metalloxiden, Carbonaten und Kombinationen davon umfasst.

12. Verfahren nach Anspruch 11, wobei sich der Schwefeloxidabsorber (104) in mindestens einer von einer porösen Struktur oder einer Wabenstruktur befindet.

13. Verfahren nach einem der Ansprüche 10 bis 12, wobei das aktive Element ferner Aluminiumoxid umfasst.

## Revendications

1. Dispositif (100) de détection de monoxyde de carbone (CO), comprenant :
un dispositif d'échantillonnage (102) pour échantillonner du gaz de combustion, le dispositif d'échantillonnage (102) comprenant un absorbeur d'oxydes de soufre (104) pour absorber les oxydes de soufre dans le gaz de combustion échantillonné ;
un détecteur de CO (115) couplé au dispositif d'échantillonnage (102) pour recevoir le gaz de combustion échantillonné après que les oxydes de soufre ont été absorbés, le détecteur de CO (115) comprenant un élément actif comprenant du platine ; et
un dispositif de chauffage (120) adapté pour réguler le détecteur de CO (115) à une température supérieure à la fois à une température de décomposition des sulfates au-dessus de laquelle une vitesse de décomposition des sulfates est supérieure à une vitesse de dépôt de sulfates et une température de combustion du coke de charbon au-dessus de laquelle une vitesse de combustion du coke de charbon est supérieure à une vitesse de dépôt de coke de charbon.

2. Dispositif de détection de CO selon la revendication 1, dans lequel l'absorbeur d'oxydes de soufre (104) est choisi parmi les hydroxydes métalliques, les oxydes métalliques, les carbonates et les combinaisons de ceux-ci.

3. Dispositif de détection de CO selon la revendication 1 ou 2, dans lequel le dispositif d'échantillonnage (102) comprend une conduite (103) pour transférer le gaz de combustion échantillonné jusqu'au détecteur de CO (115) et l'absorbeur d'oxydes de soufre (104) est disposé dans la conduite (103).

4. Dispositif de détection de CO selon la revendication 1 ou 2, dans lequel le dispositif d'échantillonnage (102) comprend une conduite (103) pour transférer le gaz de combustion échantillonné jusqu'au détecteur de CO (115) et l'absorbeur d'oxydes de soufre (104) est disposé dans un récipient (106) raccordé entre deux sections de la conduite (103).

5. Dispositif de détection de CO selon la revendication 4, dans lequel le récipient (106) a une taille radiale sensiblement identique à celle de la conduite (103) dans une direction sensiblement perpendiculaire à une direction d'écoulement du gaz de combustion échantillonné.

6. Dispositif de détection de CO selon la revendication 4, dans lequel le récipient (206) a une taille radiale supérieure à celle de la conduite (203) dans une direction sensiblement perpendiculaire à une direction d'écoulement du gaz de combustion échantillonné.

7. Dispositif de détection de CO selon une quelconque revendication précédente, dans lequel l'absorbeur d'oxydes de soufre (104) est situé dans une structure poreuse.

8. Dispositif de détection de CO selon la revendication 7, dans lequel l'absorbeur d'oxydes de soufre (104) est situé dans une structure en nid d'abeilles.

9. Dispositif de détection de CO selon une quelconque revendication précédente, dans lequel l'élément actif comprend également de l'oxyde d'aluminium.

10. Procédé, comprenant :
l'échantillonnage de gaz de combustion provenant d'une chaudière de combustion de combustible (101) ;
l'absorption des oxydes de soufre dans le gaz de combustion échantillonné ; et ensuite
la détection d'un niveau équivalent de monoxyde de carbone dans le gaz de combustion échantillonné avec un détecteur de CO (115) tel que décrit dans la revendication 1, le détecteur de CO (115) fonctionnant à une température supérieure à la fois à une température de décomposition des sulfates au-dessus de laquelle une vitesse de décomposition des sulfates est supérieure à une vitesse de dépôt de sulfates et une température de combustion du coke de charbon au-dessus de laquelle une vitesse de combustion du coke de charbon est supérieure à une vitesse de dépôt de coke de charbon.

11. Procédé selon la revendication 10, dans lequel l'absorption des oxydes de soufre comprend l'utilisation d'un absorbeur d'oxydes de soufre (104) choisi parmi les hydroxydes métalliques, les oxydes métalliques, les carbonates et les combinaisons de ceux-ci.

12. Procédé selon la revendication 11, dans lequel l'absorbeur d'oxydes de soufre (104) est situé dans une structure poreuse et/ou une structure en nid d'abeilles.

13. Procédé selon l'une quelconque des revendications 10 à 12, dans lequel l'élément actif comprend également de l'oxyde d'aluminium.
